(19) **Europäisches Patentamt / European Patent Office / Office européen des brevets**

(11) **EP 3 440 995 B1**

(12) **EUROPEAN PATENT SPECIFICATION**

(45) Date of publication and mention of the grant of the patent:
**16.06.2021 Bulletin 2021/24**

(21) Application number: **17782512.2**

(22) Date of filing: **14.04.2017**

(51) Int Cl.:
*A61B 5/021* (2006.01)　　*A61B 5/00* (2006.01)
*A61B 5/022* (2006.01)　　*A61B 5/08* (2006.01)
*A61B 5/11* (2006.01)　　*A61B 5/145* (2006.01)

(86) International application number:
**PCT/JP2017/015280**

(87) International publication number:
**WO 2017/179699 (19.10.2017 Gazette 2017/42)**

(54) **BIOLOGICAL INFORMATION ANALYSIS DEVICE, SYSTEM, AND PROGRAM**

VORRICHTUNG, SYSTEM UND PROGRAMM ZUR ANALYSE BIOLOGISCHER INFORMATIONEN

DISPOSITIF, SYSTÈME ET PROGRAMME D'ANALYSE D'INFORMATIONS BIOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.04.2016 JP 2016082463**

(43) Date of publication of application:
**13.02.2019 Bulletin 2019/07**

(73) Proprietors:
- **Omron Corporation**
  **Kyoto-shi, Kyoto 600-8530 (JP)**
- **Omron Healthcare Co., Ltd.**
  **Muko-shi, Kyoto 617-0002 (JP)**

(72) Inventors:
- **NAKAJIMA, Hiroshi**
  **Kyoto-shi**
  **Kyoto 600-8530 (JP)**
- **WADA, Hirotaka**
  **Kyoto-shi**
  **Kyoto 600-8530 (JP)**
- **TSUCHIYA, Naoki**
  **Kyoto-shi**
  **Kyoto 600-8530 (JP)**
- **KASAI, Masaaki**
  **Kyoto-shi**
  **Kyoto 600-8530 (JP)**
- **KAN, Eriko**
  **Kyoto-shi**
  **Kyoto 600-8530 (JP)**
- **UENOYAMA, Toru**
  **Tokyo 108-0075 (JP)**

- **OBAYASHI, Keiichi**
  **Kyoto-shi**
  **Kyoto 600-8530 (JP)**
- **KOKUBO, Ayako**
  **Kyoto-shi**
  **Kyoto 600-8530 (JP)**
- **OTA, Yuya**
  **Kyoto-shi**
  **Kyoto 600-8530 (JP)**
- **SHIGA, Toshikazu**
  **Muko-shi**
  **Kyoto 617-0002 (JP)**
- **KUWABARA, Mitsuo**
  **Muko-shi**
  **Kyoto 617-0002 (JP)**
- **SATO, Hironori**
  **Muko-shi**
  **Kyoto 617-0002 (JP)**
- **MIYAGAWA, Ken**
  **Muko-shi**
  **Kyoto 617-0002 (JP)**
- **TSUTSUMI, Masakazu**
  **Muko-shi**
  **Kyoto 617-0002 (JP)**

(74) Representative: **Isarpatent**
**Patent- und Rechtsanwälte Behnisch Barth Charles**
**Hassa Peckmann & Partner mbB**
**Friedrichstrasse 31**
**80801 München (DE)**

(56) References cited:
**WO-A1-2018/017425　　JP-A- 2004 261 452**

JP-A- 2005 021 619    JP-A- 2005 237 472        US-A1- 2014 276 123    US-A1- 2015 196 209
JP-A- 2005 532 111    JP-A- 2008 086 568
JP-A- 2011 189 080    US-A1- 2007 021 673

**Description**

TECHNICAL FIELD

The present invention relates to a biological information analysis device, system, and program.

RELATED ART

There is a known technology for measuring changes in the internal pressure of a radial artery and recording the shape of a pressure pulse wave (blood pressure waveform). Patent Document 1 (JP 2008-61824A) discloses that a blood pressure waveform is measured using a tonometry method, and pieces of information such as an AI (Augmentation Index) value, a pulse wave period, a baseline fluctuation rate, sharpness, and an ET (Ejection Time) are acquired from the blood pressure waveform. Also, Patent Document 2 (JP 2005-532111A) discloses that a blood pressure waveform is measured using a wristwatch-type blood pressure meter, in which a mean arterial pressure, a mean systolic pressure, a mean diastolic pressure, a mean systolic pressure indicator, and a mean diastolic pressure indicator are calculated from the blood pressure waveform, and an alert is output when any of these values deviates from a reference value.

US 2014/276,123 A1 discloses a biological information analysis device comprising: an indicator extraction unit configured to extract, from data regarding blood pressure waveforms consecutively measured by a sensor configured to be worn on a body part of a user and to be capable of non-invasively measuring a blood pressure waveform for each heartbeat, an indicator that indicates an event occurrence risk, which is the risk of an event occurring due to a change in blood pressure, based on characteristics of the blood pressure waveforms; and a processing unit configured to perform processing that is based on the indicator thus extracted.

A similar biological information analysis device is known from US 2015/196 209 A1.

US 2007/0021673 A1 discloses a method and system for cardiovascular system prognosis. The method comprises pulse wave signals during rapid excitation of the cardio vascular system, analyzing the measured signals and computing indicators reflecting a response to said excitation.

SUMMARY OF THE INVENTION

PROBLEM TO BE SOLVED BY THE INVENTION

It has been pointed out that there is the possibility of obstructive sleep apnea (OSA) and excessive exercise increasing the risk of a cardiovascular event occurring. However, conventionally, there is no effective means for detecting, in real time, an increase in the risk of such an event occurring.

The inventors of the present invention have worked hard to develop a blood pressure measurement device that can accurately measure an ambulatory blood pressure waveform for each heartbeat, and to put such a device into practical use. Through experiments performed on subjects during the development phase, the inventors have found that various kinds of useful information can be extracted from data regarding ambulatory blood pressure waveforms that have been consecutively measured. For example, although conventional blood pressure meters can only acquire information regarding blood pressure, it has become more apparent that various kinds of information related to the body of a user (e.g. information regarding the functions/states of respiratory organs and circulatory organs), in addition to information related to blood pressure, can be extracted by accurately and non-invasively monitoring ambulatory blood pressure waveforms taken every heartbeat.

Therefore, the present invention aims to provide a novel technology for detecting, in real time, an increase in an event occurrence risk.

MEANS FOR SOLVING THE PROBLEMS

To achieve the above-described aim, the present invention provides a biological information analysis device according to claim 1.

A biological information analysis device according to the present invention is a biological information analysis device including: an indicator extraction unit configured to extract, from data regarding blood pressure waveforms consecutively measured by a sensor configured to be worn on a body part of a user and to be capable of non-invasively measuring a blood pressure waveform for each heartbeat, an indicator that indicates an event occurrence risk, which is the risk of an event occurring due to a change in blood pressure, based on characteristics of the blood pressure waveforms; and a processing unit configured to perform processing that is based on the indicator thus extracted.

With this configuration, it is possible to detect, in real time, an increase in the risk of an event occurring due to a change in blood pressure, by monitoring a blood pressure waveform. Furthermore, the blood pressure waveform can be non-invasively measured. Therefore, this configuration is easy for the user, and places less physical or psychological burden on the user.

According to the invention the indicator extraction unit is configured to calculate the indicator based on an AI (Augmentation Index) and/or a BRS (Baroreflex sensitivity), which are characteristics of a blood pressure waveform. It is known that the hardness of blood vessels and the ability to regulate blood pressure are relevant to the occurrence of a cardiovascular event. An AI is a characteristic amount that indicates the hardness of blood vessels, and a BRS is a characteristic amount that indicates the ability to regulate blood pressure. Therefore, by using either one of or both the AI and the BRS, it is possible to evaluate the risk of a cardiovascular event occurring, with high reliability.

According to the invention the indicator extraction unit is configured to calculate the indicator based on a difference between an AI of a measured blood pressure waveform and a reference AI and/or a difference between a BRS of a measured blood pressure waveform and a reference BRS. By evaluating a deviation from the reference value, it is possible to easily calculate the magnitude of a risk with high reliability.

According to an alternative example which is not part of the present invention, the indicator extraction unit may be configured to calculate the indicator based on characteristics related to AI distribution and/or BRS distribution of blood pressure waveforms corresponding to a plurality of heartbeats. For example, it is preferable that the characteristics related to distribution include a mean value, and a standard deviation or dispersion. In this way, by using characteristics related to AI distribution and BRS distribution of blood pressure waveforms corresponding to a plurality of heartbeats, it is possible to increase robustness against measurement noise in blood pressure waveforms, and improve reliability when estimating an event occurrence risk.

According to this example, it is preferable that the biological information analysis device further includes: a case database in which characteristics related to AI distribution and/or BRS distribution corresponding to a plurality of heartbeats are registered for each of a plurality of cases, wherein the indicator extraction unit is configured to evaluate a degree of similarity between characteristics related to AI distribution and/or BRS distribution corresponding to a plurality of heartbeats of the user and characteristics of the plurality of cases registered in the case database, and calculate the indicator based on the result of evaluation. In this way, by evaluating the degree of similarity with a plurality of pieces of case data, it is possible to further improve reliability and objectivity when estimating the event occurrence risk.

According to an embodiment of the invention, the indicator extraction unit may be configured to predict a change in blood pressure based on characteristics of a blood pressure waveform of the user measured at the current point in time, assuming that a surge in blood pressure occurs at the current point in time, and calculate the indicator based on the result of prediction. With this method, it is possible to detect an increase in the risk of an event occurring due to a surge in blood pressure.

It is preferable that the indicator extraction unit is configured to predict a change in blood pressure based on a SBP (Systolic Blood Pressure), an AI (Augmentation Index), and a BRS (Baroreflex sensitivity), which are characteristics of a blood pressure waveform, assuming that a surge in blood pressure occurs at the current point in time. An AI is a characteristic amount that indicates the hardness of blood vessels, and a BRS is a characteristic amount that indicates the ability to regulate blood pressure. Therefore, by using both the AI and the BRS, it is possible to predict a change in blood pressure, from the SBP at the current point in time, with high reliability.

It is preferable that the processing unit is configured to perform processing to provide information indicating that the event occurrence risk has increased, upon detecting an increase in the event occurrence risk based on the indicator. As result, the user can promptly notice an increase in the risk and take countermeasures before an event occurs.

The present invention further provides a method in accordance with claim 7, which may be carried out by a device as described above, or by a program that causes a computer to execute such a method, or a computer-readable recording medium on which such a program is recorded in a non-transitory manner.

EFFECTS OF THE INVENTION

According to the present invention, it is possible to provide a novel technology for detecting, in real time, an increase in the event occurrence risk.

BRIEF DESCRIPTION OF THE DRAWINGS

FIG. 1 shows a schematic external configuration of a biological information analysis system 10.
FIG. 2 is a block diagram showing a hardware configuration of the biological information analysis system 10.
FIG. 3 is a cross-sectional view schematically showing a configuration of a blood pressure measurement unit 20 and a state in which measurement is performed.
FIG. 4 shows a blood pressure waveform that is measured by the blood pressure measurement unit 20.

FIG. 5 is a block diagram illustrating processing that is performed by a biological information analysis device 1.

FIG. 6 shows a waveform (a blood pressure waveform) of a pressure pulse wave from a radial artery corresponding to one heartbeat.

FIG. 7 is a flowchart for event occurrence risk calculation processing according to Example 1.

FIG. 8 is a conceptual diagram showing an AI risk according to Example 1.

FIG. 9 shows an example of an information output screen according to Example 1.

FIG. 10 is another example of a conceptual diagram showing an AI risk according to Example 1.

FIG. 11 is a flowchart for event occurrence risk calculation processing according to Example 2.

FIG. 12 illustrates surge shape estimation processing according to Example 2.

FIG. 13 shows an example of an information output screen according to Example 2.

EMBODIMENTS OF THE INVENTION

The following describes a preferred embodiment of the present invention with reference to the drawings. Note that the following descriptions of components may be modified as appropriate depending on the configuration of a device to which the present invention is applied, and on various conditions, and the scope of the present invention is not intended to be limited to the following descriptions.

Biological Information Analysis System

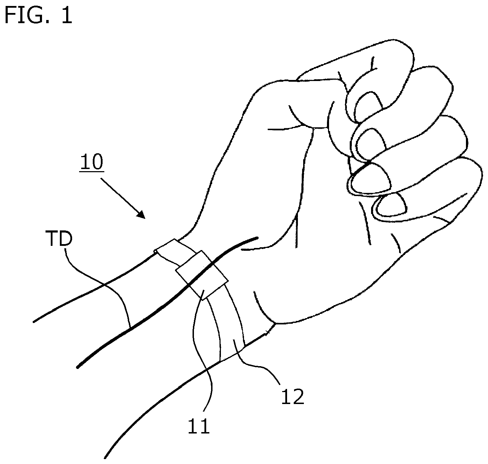

FIG. 1 shows a schematic external configuration of a biological information analysis system 10 according to an embodiment of the present invention. FIG. 1 shows a state in which the biological information analysis system 10 is worn on the left wrist. The biological information analysis system 10 includes a main body 11 and a belt 12 that is fixed to the main body 11. The biological information analysis system 10 is a so-called wearable device, and is worn such that the main body 11 is in contact with the skin on the palm side of the wrist, and the main body 11 is located over a radial artery TD that lies beneath the skin. Although the device is configured to be worn on the radial artery TD in the present embodiment, the device may be configured to be worn on another superficial artery.

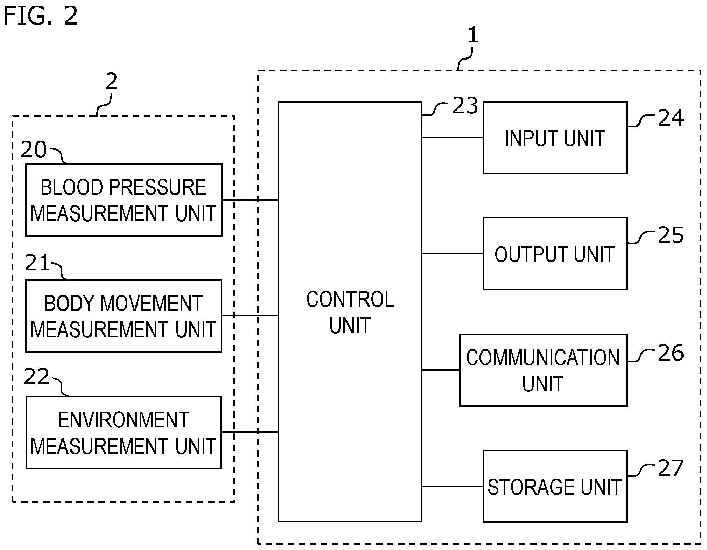

FIG. 2 is a block diagram showing a hardware configuration of the biological information analysis system 10. In general, the biological information analysis system 10 includes a measurement unit 2 and the biological information analysis device 1. The measurement unit 2 is a device that performs measurement to acquire information that is used to analyze biological information, and includes a blood pressure measurement unit 20, a body movement measurement unit 21, and an environment measurement unit 22. However, note that the configuration of the measurement unit 2 is not limited to that shown in FIG. 2. For example, a unit that measures biological information other than blood pressure or a body movement (e.g. body temperature, blood-sugar level, or brain waves) may be added. Also, any unit that is not used in the example described below is not an essential component, and may be omitted from the biological information analysis system 10. The biological information analysis device 1 is a device that analyzes biological information based on information acquired from the measurement unit 2, and includes a control unit 23, an input unit 24, an output unit 25, a communication unit 26, and a storage unit 27. The units 20 to 27 are connected to each other so that signals can be exchanged between them via a local bus or other signal lines. The biological information analysis system 10 also includes a power supply (a battery), which is not shown.

The blood pressure measurement unit 20 measures a pressure pulse wave from the radial artery TD by using a tonometry method. The tonometry method is for forming a flat area in the artery TD by pressing the artery from the skin with appropriate pressure, adjusting the balance between the internal pressure and the external pressure of the artery, and non-invasively measuring the pressure pulse wave using a pressure sensor.

The body movement measurement unit 21 includes a tri-axis acceleration sensor, and measures the movement of the user's body (body movement) using this sensor. The body movement measurement unit 21 may include a circuit that converts the format of an output from the tri-axis acceleration sensor into a format that is readable to the control unit 23.

The environment measurement unit 22 measures environmental information that may affect mental and physical conditions of the user (in particular the blood pressure). The environment measurement unit 22 may include, for example, an atmospheric temperature sensor, a humidity sensor, an illuminance sensor, an altitude sensor, a position sensor, and so on. The environment measurement unit 22 may include a circuit that converts the format of outputs from these sensors and so on into a format that is readable to the control unit 23.

The control unit 23 performs various kinds of processing, such as controlling each unit of the biological information analysis system 10, acquiring data from the measurement unit 2, storing the acquired data in the storage unit 27, processing and analyzing data, and inputting and outputting data. The control unit 23 includes a hardware processor (hereinafter referred to as the "CPU") a ROM (Read Only Memory), a RAM (Random Access Memory), and so on. Processing that is performed by the control unit 23, which will be described later, is realized by the CPU reading and executing a program stored in the ROM or the storage unit 27. The RAM functions as a work memory that is used by the control unit 23 when performing various kinds of processing. Although acquisition of data from the measurement unit 2 and the storing of data in the storage unit 27 are performed by the control unit 23 in the present embodiment, it is possible to employ a configuration in which the measurement unit 2 directly stores (writes) data in the storage unit 27.

Each of the constituent components of the embodiment such as a measurement unit, an indicator extraction unit, a processing unit, a determination unit, a risk database, an input unit, an output unit, a case database, and so on may be implemented as pieces of hardware in the biological information analysis system 10. The indicator extraction unit, the processing unit, and the determination unit may receive an executable program stored in the storage unit 27, and execute the program. The indicator extraction unit, the processing unit, and the determination unit may receive data from the blood pressure measurement unit 20, the body movement measurement unit 21, the environment measurement unit 22, the input unit 24, the output unit 25, the communication unit 26, the storage unit 27, and so on as required. Databases such as the risk database and the case database may be implemented using the storage unit 27 and so on, and store pieces of information that are arranged such that a data search and data accumulation can be easily performed. Here, for example, the configuration, operations, and so on of the biological information analysis system 10 are disclosed in JP 2016-082069A. Also, the configuration, operations, and so on of the blood pressure measurement unit are disclosed in JP 2016-087003A.

The input unit 24 provides an operation interface for the user. For example, an operation button, a switch, a touch panel, and so on may be used.

The output unit 25 provides an interface that outputs information to the user. For example, a display device (such as a liquid crystal display) that outputs information using images, an audio output device or a beeper that outputs information using audio, an LED that outputs information by blinking, a vibration device that outputs information by vibrating, and so on may be used.

The communication unit 26 performs data communication with another device. Any data communication method such as a wireless LAN or Bluetooth (registered trademark) may be used.

The storage unit 27 is a storage medium that can store data and from which data can be read out, and stores programs that are to be executed by the control unit 23, pieces of measurement data acquired from the measurement units, and various kinds of data acquired by processing the pieces of measurement data, and so on. The storage unit 27 is a medium that accumulates pieces of information that are to be stored, through an electrical, magnetic, optical, mechanical, or chemical action. For example, a flash memory is used. The storage unit 27 may be a portable unit such as a memory card, or built into the biological information analysis system 10.

At least one unit or all units out of the body movement measurement unit 21, environment measurement unit 22, the control unit 23, the input unit 24, the output unit 25, and the storage unit 27 may be configured as a device that is separate from the main body 11. That is, as long as the blood pressure measurement unit 20 and the main body 11 that incorporates a circuit that controls the blood pressure measurement unit 20 are configured to be wearable on a wrist, the configurations of other units can be freely designed. If this is the case, the main body 11 cooperates with another unit via the communication unit 26. Various configurations can be conceived of. For example, the functions of the control unit 23, the input unit 24, and the output unit 25 may be realized using a smartphone application, and required data may be acquired from an activity monitor that has the functions of the body movement measurement unit 21 and the environment measurement unit 22. Also, a sensor that measures biological information other than blood pressure may be provided. For example, a sleep sensor, a pulse oximeter (an $SpO_2$ sensor), a respiration sensor (a flow sensor), a blood-sugar level sensor, and the like may be combined.

Although the sensor (the blood pressure measurement unit 20) that measures blood pressure and the component (including the control unit 23 and so on) that performs processing to analyze blood pressure waveform data are provided in one device in the present embodiment, they may be provided in separate members. In the present embodiment, the component (including the control unit 23 and so on) that performs processing to analyze biological information is referred to as a biological information analysis device, and the device that includes the combination of the measurement unit and the biological information analysis device is referred to as a biological information analysis system. However, these names are given for descriptive purposes, and the measurement unit and the component that performs processing to analyze biological information may be referred to as a biological information analysis device as a whole, or other names may be used.

Measurement of Blood Pressure Waveform

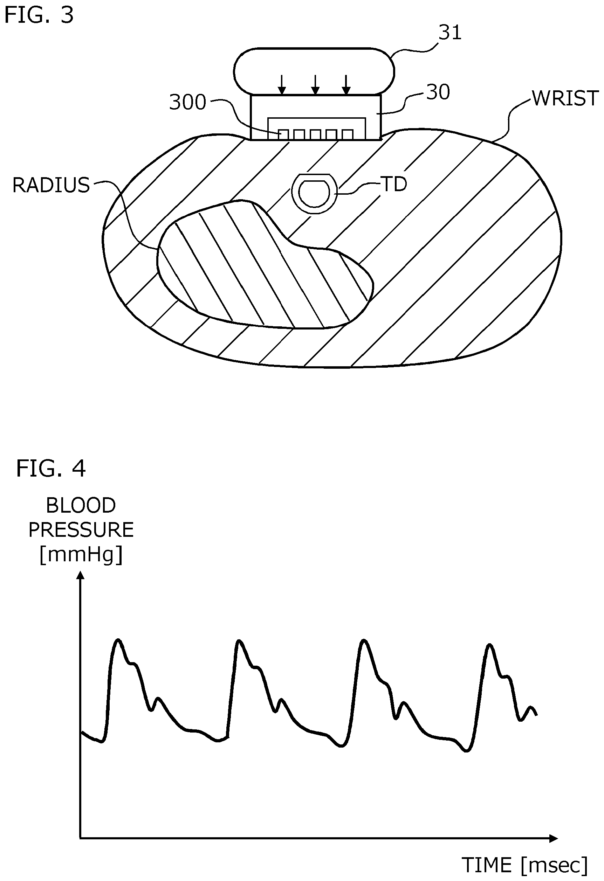

FIG. 3 is a cross-sectional view schematically showing the configuration of the blood pressure measurement unit 20 and a state in which measurement is performed. The blood pressure measurement unit 20 includes a pressure sensor 30 and a pressurizing mechanism 31 for pressing the pressure sensor 30 against a wrist. The pressure sensor 30 includes a plurality of pressure detection elements 300. The pressure detection elements 300 detect pressure and convert the pressure into an electrical signal. For example, elements that utilize a piezoresistive effect may be preferably used. The pressurizing mechanism 31 includes, for example, an air bag and a pump that adjusts the internal pressure of the air bag. As a result of the control unit 23 controlling the pump to increase the internal pressure of the air bag, the air bag expands and the pressure sensor 30 is pressed against the surface of the skin. Note that the pressurizing mechanism 31 may be any mechanism as long as it can adjust the pressing force of the pressure sensor 30 applied to the surface of the skin, and is not limited to a mechanism that uses an air bag.

Upon the biological information analysis system 10 being worn on a wrist and activated, the control unit 23 controls the pressurizing mechanism 31 of the blood pressure measurement unit 20 to keep the pressing force of the pressure sensor 30 in an appropriate state (a tonometry state). Then, pressure signals detected by the pressure sensor 30 are sequentially acquired by the control unit 23. Pressure signals acquired from the pressure sensor 30 are generated by digitizing analogue physical amounts (e.g. voltage values) output by the pressure detection elements 300, through an A/D converter circuit or the like that employs a well-known technology. Preferable analogue values such as current values or resistance values may be employed as the analogue physical amounts, depending on the type of the pressure detection elements 300. Signal processing such as the aforementioned A/D conversion may be performed using a predetermined circuit provided in the blood pressure measurement unit 20, or performed by another unit (not shown) provided between the blood pressure measurement unit 20 and the control unit 23. Each pressure signal acquired by the control unit 23 corresponds to an instantaneous value of the internal pressure of the radial artery TD. Therefore, it is possible to acquire time-series data regarding blood pressure waveforms by acquiring pressure signals with time granularity and continuity that make it possible to ascertain a blood pressure waveform for each heartbeat. The control unit 23 stores the pressure signals sequentially acquired from the pressure sensor 30, in the storage unit 27, together with information regarding points in time at which the pressure signals were measured. The control unit 23 may store the acquired pressure signals in the storage unit 27 without change, or store the pressure signals in the storage unit 27 after performing required signal processing on the pressure signals. Required signal processing includes, for example, processing that is performed to calibrate each pressure signal such that the amplitude of the pressure signal matches the blood pressure value (e.g. the brachial blood pressure), processing that is performed to reduce or remove noise in each pressure signal, and so on.

FIG. 4 shows a blood pressure waveform measured by the blood pressure measurement unit 20. The horizontal axis indicates time and the vertical axis indicates blood pressure. Although the sampling frequency may be set to any value, it is preferably set to be no less than 100 Hz so that characteristics of the shape of a waveform corresponding to one heartbeat can be reproduced. Typically, the period of one heartbeat is approximately one second, and therefore approximately one hundred or more data points can be acquired on a waveform corresponding to one heartbeat.

The blood pressure measurement unit 20 according to the present embodiment is advantageous in terms of the following.

The blood pressure measurement unit 20 can measure a blood pressure waveform for each heartbeat. As a result, it is possible to acquire various indicators related to blood pressure, the state of the heart, cardiovascular risks, and so on, based on the characteristics of the shape of the blood pressure waveform. In addition, it is possible to monitor for instantaneous values of blood pressure. Therefore, it is possible to instantaneously detect a blood pressure surge (a sudden rise in the blood pressure value), and to detect changes in blood pressure and irregularities in a blood pressure waveform that may occur in a very short period of time (corresponding to one to several heartbeats) without missing them.

As a portable blood pressure meter, a blood pressure meter that is to be worn on a wrist or an upper arm and employs an oscillometric method to measure blood pressure has come into practical use. However, a conventional portable blood pressure meter can only measure the mean value of blood pressure based on changes in the internal pressure of a cuff during a period of several seconds to a dozen or so seconds corresponding to a plurality of heartbeats, and cannot acquire time-series data regarding a blood pressure waveform for each heartbeat, unlike the blood pressure measurement unit 20 according to the present embodiment.

The blood pressure measurement unit 20 can record time-series data regarding blood pressure waveforms. By acquiring time-series data regarding blood pressure waveforms, and, for example, discerning characteristics of the blood pressure waveform related to temporal changes, or performing a frequency analysis on the time-series data to extract a specific frequency component, it is possible to acquire various indicators related to blood pressure, the state of the heart, cardiovascular risks, and so on.

The device employs a portable (wearable) type configuration, and less burden is placed on the user during measurement. Therefore, continuous measurement for a long time, and even 24-hour blood pressure monitoring, can be relatively easily performed. Also, since the device is of a portable type, changes in not only blood pressure under resting conditions, but also an ambulatory blood pressure (for example, during daily life or exercise) can be measured. As a result, it is possible to grasp how blood pressure is affected by behaviours in daily life (such as sleeping, eating, commuting, working, and taking medicine) and exercise, for example.

Conventional products are types of devices that measure blood pressure under resting conditions, with an arm or a wrist fixed to a blood pressure measurement unit, and cannot measure changes in blood pressure in daily life or during exercise, unlike the biological information analysis system 10 according to the present embodiment.

The blood pressure measurement unit 20 can be easily combined or linked with other sensors. For example, it is possible to make an evaluation of a cause-effect relationship or a composite evaluation with information that can be acquired by other sensors (e.g. a body movement, environmental information such as an atmospheric temperature, biological information such as $SpO_2$ and respiration information).

Biological Information Analysis Device

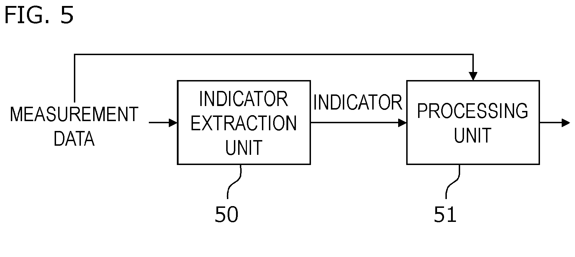

FIG. 5 is a block diagram illustrating processing that is performed by the biological information analysis device 1. As shown in FIG. 5, the biological information analysis device 1 includes an indicator extraction unit 50 and a processing unit 51. In the present embodiment, processing performed by the indicator extraction unit 50 and the processing unit 51 may be realized by the control unit 23 executing a program that is required for the processing. The program may be stored in the storage unit 27. When the control unit 23 executes the required program, the subject program stored in the ROM or storage unit 27 is loaded to the RAM. Then, the control unit 23 interprets and executes the program loaded to the RAM, using the CPU, to control each constituent component. Note that at least one or all of the processing procedures executed by the indicator extraction unit 50 and the processing unit 51 may be realized using a circuit such as an ASIC or an FPGA. Alternatively, at least one or all of the processing procedures executed by the indicator extraction unit 50 and the processing unit 51 may be realized using a computer (e.g. a smartphone, a tablet terminal, a personal computer, or a cloud server) that is separate from the main body 11.

The indicator extraction unit 50 acquires time-series data regarding blood pressure waveforms, which have been consecutively measured by the blood pressure measurement unit 20, from the storage unit 27. The indicator extraction unit 50 extracts, from the acquired time-series data regarding blood pressure waveforms, indicators that are related to characteristics of the blood pressure waveforms. Here, characteristics of a blood pressure waveform include, for example, characteristics of the shape of a blood pressure waveform corresponding to one heartbeat, temporal changes in a blood pressure waveform, and frequency components of a blood pressure waveform. However, characteristics of a blood pressure waveform are not limited to those listed above. The extracted indicators are output to the processing unit 51. There are various characteristics and indicators regarding a blood pressure waveform, and the characteristics and indicators that are to be extracted may be designed or selected as appropriate according to the purpose of processing that is to be performed by the processing unit 51. Characteristics and indicators that can be extracted from measurement data regarding blood pressure waveforms according to the present embodiment will be described later in detail.

When obtaining indicators, the indicator extraction unit 50 may use measurement data that has been acquired by the body movement measurement unit 21 and/or measurement data that has been acquired by the environment measurement unit 22, in addition to measurement data regarding blood pressure waveforms. Also, although not shown in the drawings, pieces of measurement data that have been acquired by a sleep sensor, an $SpO_2$ sensor, a respiration sensor (a flow sensor), a blood-sugar level sensor, and the like may be combined with one another. By performing complex analysis on a plurality of kinds of measurement data acquired by a plurality of sensors, it is possible to perform more advanced information analysis of a blood pressure waveform. For example, it is possible to classify pieces of data regarding blood pressure waveforms according to states of the user, such as a resting state and a moving state, a state when an atmospheric temperature is high and a state when it is low, a light sleep state and a deep sleep state, a breathing state and an apnea state, and so on. Alternatively, it is possible to extract information regarding the influence of body movement, an activity amount, activity intensity, a change in an atmospheric temperature, apnea, the user's breathing, etc. on blood pressure, and thus evaluate the cause-effect relationship, the correlation, etc. between pieces of measurement data. Note that examples of apnea include obstructive sleep apnea, central sleep apnea, and mixed sleep apnea.

The processing unit 51 receives the indicators extracted by the indicator extraction unit 50. The processing unit 51 performs processing that is based on the received indicators. Various kinds of processing can be conceived of as processing that is based on the indicators. For example, the processing unit 51 may provide the values of the extracted indicators or changes in the values to a user, a doctor, a public health nurse, or the like to prompt the utilization of the indicators in the fields of health care, treatment, health guidance, and so on. Alternatively, the processing unit 51 may provide guidelines for health maintenance or risk mitigation. Furthermore, when an increase in the event occurrence risk is detected or predicted, the processing unit 51 may inform the user or his/her doctor, or perform control to prevent the user from performing an action that places a burden on his/her heart and so on, or to prevent a cardiovascular event from occurring.

Information Acquired from Blood Pressure Waveform

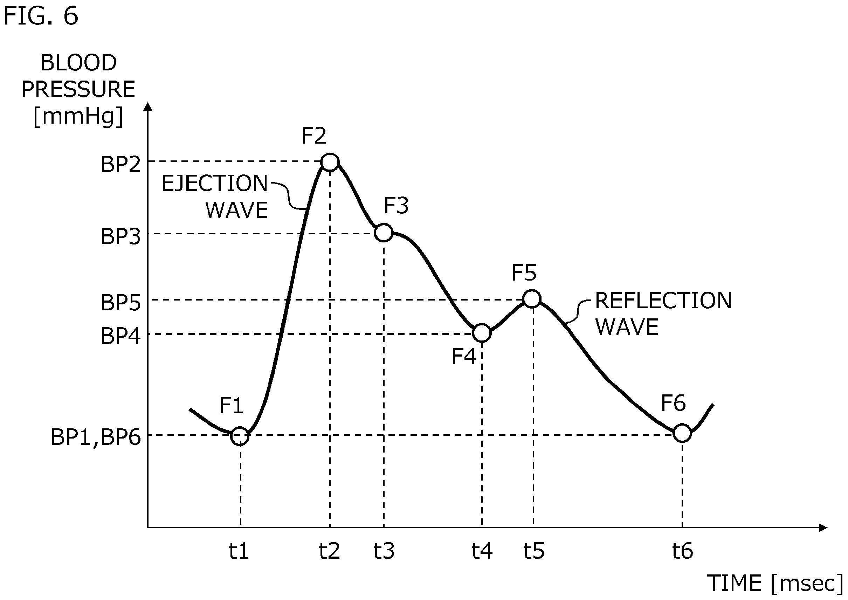

FIG. 6 shows a waveform (a blood pressure waveform) of a pressure pulse wave from a radial artery corresponding to one heartbeat. The horizontal axis indicates time t (msec) and the vertical axis indicates blood pressure BP (mmHg).

A blood pressure waveform is the waveform of a composite wave constituted by an "ejection wave" that is generated when the heart contracts and pumps out blood, and a "reflection wave" that is generated when an ejection wave is reflected at a branch point of a peripheral vessel or an artery. The following shows examples of characteristic points that can be extracted from a blood pressure waveform corresponding to one heartbeat.

- A point F1 is the rising point of the pressure pulse wave. The point F1 corresponds to the ejection start point of the heart, i.e. the point at which the aortic valve opens.
- A point F2 is a point at which the amplitude (the pressure) of the ejection wave is at the maximum (a first peak).
- A point F3 is an inflection point that appears midway in a drop in the ejection wave, due to a reflection wave being superimposed.
- A point F4 is the minimum point, which appears between the ejection wave and the reflection wave, and is also referred to as a notch. This point corresponds to the point at which the aortic valve closes.
- A point F5 is the peak of the reflection wave (a second peak), which appears after the point F4.
- A point F6 is the end point of one heartbeat, and corresponds to the ejection start point of the next heartbeat, i.e. the start point of the next heartbeat.

The indicator extraction unit 50 may use any algorithm to detect the above-described characteristic points. For example, the indicator extraction unit 50 may perform computations to obtain an nth order differential waveform of a blood pressure waveform, and detect the zero-crossing points to extract the characteristic points (the inflection points) of the blood pressure waveform (the points F1, F2, F4, F5, and F6 can be detected from the first order differential waveform, and the point F3 can be detected from the second order differential waveform or the fourth order differential waveform). Alternatively, the indicator extraction unit 50 may read out, from the storage unit 27, a waveform pattern on which the characteristic points have been arranged in advance, and perform fitting of the waveform pattern to the target blood pressure waveform to specify the respective positions of the characteristic points.

The indicator extraction unit 50 performs computations based on time t and pressure BP of each of the above-described characteristic points F1 to F6, and can thus obtain various kinds of information (values, characteristic amounts, indicators, etc.) from the blood pressure waveform of one heartbeat. The following are typical examples of information that can be acquired from a blood pressure waveform. Note that tx and BPx respectively represent time and blood pressure corresponding to a characteristic point Fx.

- Pulse Wave Interval (Period of Heartbeat) TA = t6 - t1
- Heart Rate PR = 1/TA
- Pulse Wave rising Time UT = t2 -t1
- Systole TS = t4 - t1
- Diastole TD = t6 - t4
- Reflection Wave Delay Time = t3 - t1
- Maximum Blood Pressure (Systolic Blood Pressure) SBP = BP2
- Minimum Blood Pressure (Diastolic Blood Pressure) DBP = BP1
- Mean Blood Pressure MAP = (Area of Blood Pressure Waveform from t1 to t6)/Period of Heartbeat TA
- Mean Blood Pressure during Systole = (Area of Blood Pressure Waveform from t1 to t4)/Systole TS
- Mean Blood Pressure during Diastole = (Area of Blood Pressure Waveform from t4 to t6)/Diastole TS
- Pulse Pressure PP = Maximum Blood Pressure SBP - Minimum Blood Pressure DBP
- Late Systolic Pressure SBP2 = BP3
- AI (Augmentation Index) = (Late Systolic Pressure SBP2 - Minimum Blood Pressure DBP)/Pulse Pressure PP

Basic statistics of these pieces of information (values, characteristic amounts, and indicators) can also be used as indicators. Basic statistics include, for example, representative values (a mean value, a median value, a mode value, the maximum value, the minimum value, and so on) and the degree of scatter (dispersion, a standard deviation, a coefficient of variation, and so on). Temporal changes in these pieces of information (values, characteristic values, and indicators) can also be used as indicators.

In addition, the indicator extraction unit 50 can also acquire an indicator called BRS (Baroreflex Sensitivity) by performing computations on pieces of beat information. This indicator indicates the ability to adjust blood pressure to be constant. Examples of methods for calculating the indicator include a spontaneous sequence method. This is a method for only extracting a sequence in which the maximum blood pressure SBP and the pulse wave interval TA consecutively rise or fall over the period of three or more beats in synchronization with each other, plotting the maximum blood pressure SBP and the pulse wave interval TA onto a two-dimensional plane, and defining the inclination of the regression line obtained through a least squares method as the BRS.

As described above, the use of the biological information analysis system 10 according to the present embodiment makes it is possible to acquire various kinds of information from blood pressure waveform data. However, the biological information analysis system 10 need not implement all of the functions that are required to acquire all of the kinds of information described above. The biological information analysis system 10 need only implement functions that are required to acquire necessary information, depending on the configuration of the biological information analysis system 10, who the user is, the purpose of use, the location of use, and so on. Also, each function may be provided as a program module (a piece of application software), and the biological information analysis system 10 may employ a mechanism with which a function can be added by installing a necessary program module on the biological information analysis system 10.

The following illustrates several examples, which are specific applications, of the biological information analysis system 10.

Example 1

This is an example in which a blood pressure waveform is monitored and an increase in the event occurrence risk is detected.

It is known that the hardness of blood vessels and the ability to regulate blood pressure are relevant to the occurrence of a cardiovascular event. The hardness of blood vessels is indicated by an indicator called AI (Augmentation Index). The ability to regulate blood pressure is the ability to keep the value of blood pressure within a certain range, and is indicated by an indicator called BRS (Baroreflex Sensitivity). The indicator extraction unit 50 according to the present example calculates the AI from a blood pressure waveform for each heartbeat, and calculates the BRS based on the values of systolic blood pressure SBP and the values of the pulse wave interval TA corresponding to each given heartbeat and two or more heartbeats that are immediately followed by the given heartbeat, i.e. three or more heartbeats in total. Then, the indicator extraction unit 50 calculates an indicator (referred to as an event occurrence risk) that indicates the risk of a cardiovascular event occurring due to changes in blood pressure, based on the values of the AI and the BRS.

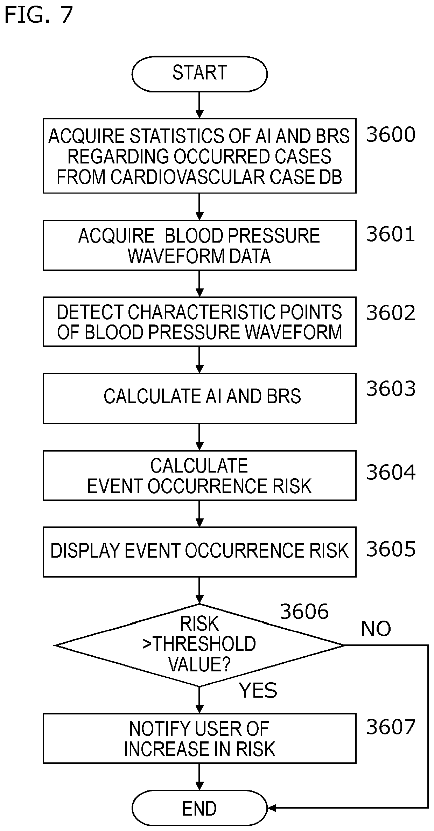

FIG. 7 shows a flowchart for processing according to the present example.

First, the indicator extraction unit 50 acquires statistics (mean values and standard deviations) of the AI and the BRS regarding cases of cardiovascular events that have actually occurred, from a case database of cardiovascular events (step 3600). The case database of cardiovascular events is a database in which information regarding a large number of cases related to cardiovascular events is registered, and is assumed to be available via the Internet, for example.

Next, the indicator extraction unit 50 reads data regarding blood pressure waveforms corresponding to the three or more most recent heartbeats from the storage unit 27 (step 3601), and detects the characteristic points F1 to F6 of a blood pressure waveform by performing characteristic point detection processing (step 3602). A specific method for performing characteristic point detection processing is as described with reference to FIG. 6. Note that the indicator extraction unit 50 may read data regarding the blood pressure waveform corresponding to the most recent one heartbeat directly from the blood pressure measurement unit 20, instead of from the storage unit 27. Also, data regarding the characteristic points F1 to F6 detected in step 3602 may be stored in the storage unit 27, and from the next time, the indicator extraction unit 50 may not perform processing in step 3602 on the same blood pressure waveform (instead, read the characteristic points F1 to F6 from the storage unit 27).

Next, the indicator extraction unit 50 calculates the AI (= (BP3-BP1)/(BP2-BP1)) based on the blood pressure value BP1 (the diastolic blood pressure DBP) at the characteristic point F1, the blood pressure value BP2 (the systolic blood pressure SBP) at the characteristic point F2, and the blood pressure value BP3 (the late systolic pressure SBP2) at the characteristic point F3. Also, the indicator extraction unit 50 calculates the BRS based on the values of the systolic blood pressure SBP and the values of the pulse wave interval TA corresponding to two or more heartbeats that are immediately followed by a given heartbeat, and the value of the SBP and the value of the TA corresponding to the given heart beat (step 3603). Thus, it is possible to obtain the values of the AI and the BRS for each heartbeat of the user.

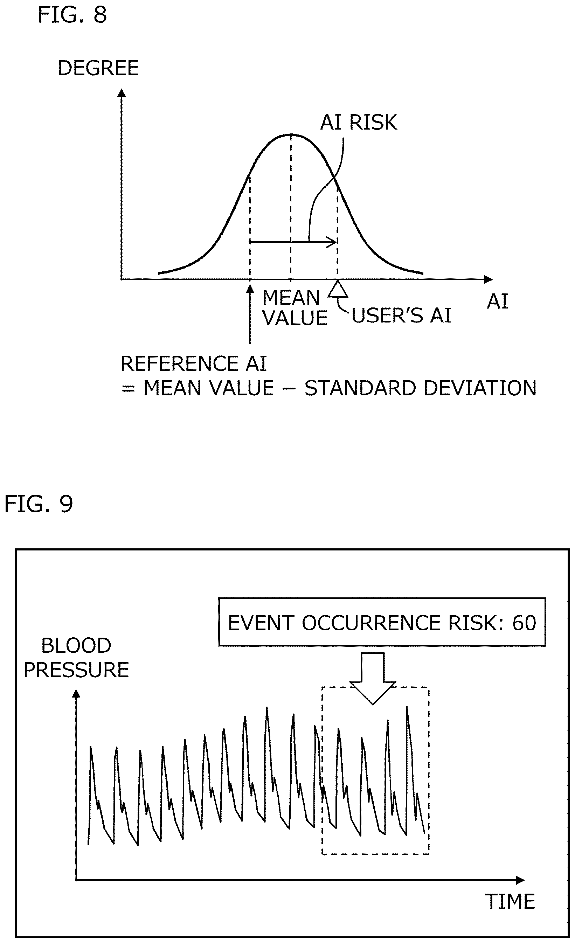

Next, the indicator extraction unit 50 calculates the event occurrence risk based on the mean value and the standard deviation of the AI and the BRS of the occurred cases, and the values of the user's AI and BRS (step 3604). Specifically, the indicator extraction unit 50 calculates the risks regarding the AI and the BRS according to the following equations, and defines the total of the AI risk and the BRS risk as the event occurrence risk. FIG. 8 shows the conceptual diagram of the AI risk.

$$\text{Event Occurrence Risk} = \text{AI Risk} + \text{BRS Risk}$$

$$\text{AI Risk} = \text{User's AI} - \text{Reference AI}$$

$$\text{Reference AI} = \text{Mean Value of AI of Occurred Cases} - \text{Standard Deviation of AI of Occurred Cases}$$

$$\text{BRS Risk} = \text{User's BRS} - \text{Reference BRS}$$

$$\text{Reference BRS} = \text{Mean Value of BRS of Occurred Cases} - \text{Standard Deviation of BRS of Occurred Cases}$$

At this time, it is preferable that the indicator extraction unit 50 performs normalization so that the AI risk and the BRS risk both range from 0 to 50. This is because such a configuration equalizes the importance of the AI and the BRS, and allows the event occurrence risk to take values ranging from 0 to 100, which makes the indicators useful.

Next, the processing unit 51 displays the event occurrence risk calculated in step 3604, on a display device (step 3605). FIG. 9 shows an example of an information output screen. In the example shown in FIG. 9, the score of the event occurrence risk and a blood pressure waveform corresponding to the score are displayed. Furthermore, if the event occurrence risk is greater than a threshold value (step 3606), the processing unit 51 informs the user of an increase in the event occurrence risk, by sounding an alarm and/or generating vibrations (step 3607). The processing performed in steps 3601 to 3607 is repeated for each heartbeat.

With the above-described configuration, it is possible to detect, in real time, an increase in the event occurrence risk. Also, when the risk has increased to a certain level, it is possible to allow the user to take countermeasures before an event occurs, by sounding the alarm and/or generating vibrations to inform the user. For example, if an increase in the risk is detected when the user is moving his/her body, the user may immediately assume a posture for rest, by sitting down, lying down, or the like. Also, if an increase in the risk caused by the occurrence of obstructive sleep apnea is detected, it is possible to prompt the user to wake up, by sounding the alarm and/or generating vibrations, or recommend the user to go to hospital.

Although two characteristic amounts, namely the AI and the BRS, are used in the above-described configuration, the risk may be calculated using only one of them. Alternatively, another characteristic amount may be used to calculate the risk.

If the cardiovascular event case database contains data regarding the AI and the BRS for each type of event (e.g. cerebral infarction, subarachnoid hemorrhage, or heart failure), the event occurrence risk may be calculated for each type of event. Also, if the cardiovascular event case database contains cholesterol values and information regarding blood (e.g. viscosity), these pieces of information may be used to calculate the event occurrence risk. A specific method is, as with the AI, etc., to define "Mean Value - Standard Deviation" of the case data as the reference value, and define the difference between the reference value and the user's measurement value as the risk.

In the above-described embodiment, the reference value is "Mean Value - Standard Deviation". However, if it is desired to make a stricter judgement, i.e. if it is desired to avoid overlooking even a very low risk, a weighting coefficient for the standard deviation may be changed, as in "Mean Value - 2 × Standard Deviation". Furthermore, if a reference that is different from the reference in the present definition is discovered as being medically useful, such a reference may be used.

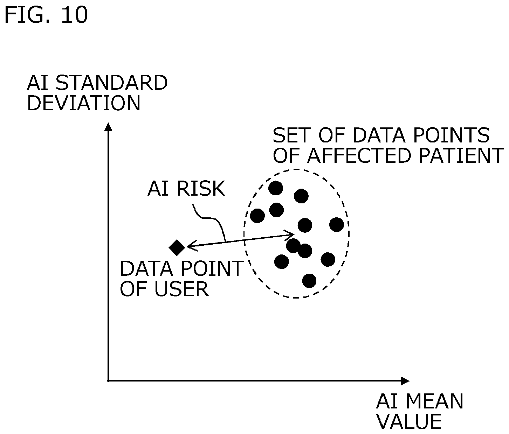

Also, according to an example which is not part of the present invention, if pieces of measurement data regarding the AI, taken a plurality of times, or statistics (a mean value, a standard deviation, dispersion, and so on) of the AI are registered in the cardiovascular event case database for each of a plurality of cases (a plurality of affected patients), the indicator extraction unit 50 may calculate the AI risk as shown in FIG. 10. That is, the indicator extraction unit 50 calculates the mean value and the standard deviation of the AI for each case (each affected patient), and represents each case in a two-dimensional space defined by AI mean value and AI deviation value. Similarly, the indicator extraction unit 50 also plots an AI mean value and an AI standard deviation obtained from blood pressure waveform data regarding a plurality of heartbeats of the user, on the two-dimensional space. Then, the indicator extraction unit 50 evaluates a degree of similarity between the set of data points of all of the cases and the data point of the user, and calculates the AI risk based on the result of evaluation (the degree of similarity). The degree of similarity may be used as it is as the AI risk, or a score that is positively correlated with the degree of similarity may be used as the AI risk. The indicator extraction unit 50 may use, as the degree of similarity, the distance between the set of data points of all of the cases and the data point of the user, for example. A shorter distance indicates a higher degree of similarity, and accordingly indicates a higher AI risk. Note that the distance between the set of data points of all of the cases and the data point of the user may be the distance between the centroid of the data points of all of the cases and the data point of the user, or the Mahalanobis distance. A similar definition can be applied to the BRS risk. Alternatively, four-dimensional space defined by the AI mean, the AI standard deviation, the BRS mean, and the BRS standard deviation may be used to obtain the risk considering both indicators.

In this way, by calculating the event occurrence risk (the risk) based on characteristics related to AI distribution and BRS distribution (a mean value, a standard deviation, dispersion, and so on) of blood pressure waveforms corresponding to a plurality of heartbeats of the user, it is possible to increase robustness against measurement noise in blood pressure waveforms, and improve reliability when estimating the event occurrence risk. Also, by evaluating the degree of similarity with a plurality of pieces of case data, it is possible to further improve reliability and objectivity when estimating the event occurrence risk. Example 2

This example, which is not part of the present invention, is also an example in which a blood pressure waveform is monitored and an increase in the event occurrence risk is detected. However, information regarding the event occurrence risk is detected using an algorithm that is different from the algorithm used in Example 1.

It is known that the hardness of blood vessels and the ability to regulate blood pressure are relevant to the occurrence of a cardiovascular event. The hardness of blood vessels is indicated by an indicator called AI (Augmentation Index). The ability to regulate blood pressure is the ability to keep the value of blood pressure within a certain range, and is indicated by an indicator called BRS (Baroreflex Sensitivity). The indicator extraction unit 50 according to the present example calculates the systolic blood pressure (SBP) and the AI from a blood pressure waveform for each heartbeat, and calculates the BRS based on the values of the systolic blood pressure SBP and the values of the pulse wave intervals TA for each given heartbeat and two or more heartbeats that are immediately followed by the given heartbeat, i.e. three or more heartbeats in total. Then, the indicator extraction unit 50 predicts "a change in blood pressure in a case where a surge in blood pressure occurs at the current point in time" based on these values, and calculates an indicator (referred to as an event occurrence risk) that indicates the risk of an event occurring due to a change in blood pressure, based on the result of prediction.

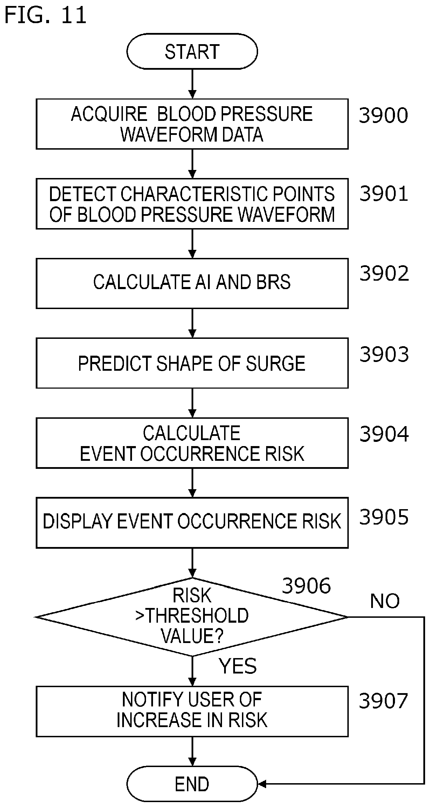

FIG. 11 shows a flowchart for processing according to the present example.

First, the indicator extraction unit 50 reads data regarding blood pressure waveforms corresponding to the three or more most recent heartbeats from the storage unit 27 (step 3900), and detects the characteristic points F1 to F6 of a blood pressure waveform by performing characteristic point detection processing (step 3901). A specific method for performing characteristic point detection processing is as described with reference to FIG. 6. Note that the indicator extraction unit 50 may read data regarding the blood pressure waveform corresponding to the most recent one heartbeat directly from the blood pressure measurement unit 20, instead of from the storage unit 27. Also, data regarding the characteristic points F1 to F6 detected in step 3901 may be stored in the storage unit 27, and from the next time, the indicator extraction unit 50 may not perform processing in step 3901 on the same blood pressure waveform (instead, read the characteristic points F1 to F6 from the storage unit 27).

Next, the indicator extraction unit 50 calculates the AI (= (BP3 - BP1)/(BP2 - BP1)) based on the blood pressure value BP1 (the diastolic blood pressure DBP) at the characteristic point F1, the blood pressure value BP2 (the systolic blood pressure SBP) at the characteristic point F2, and the blood pressure value BP3 (the late systolic pressure SBP2) at the characteristic point F3. Also, the indicator extraction unit 50 calculates the BRS based on the values of the systolic blood pressure SBP and the values of the pulse wave interval TA corresponding to two or more heartbeats that are immediately followed by a given heartbeat, and the value of the SBP and the value of the TA corresponding to the given heart beat (step 3902). Thus, it is possible to obtain the values of the SBP, the AI, and the BRS for each heartbeat of the user.

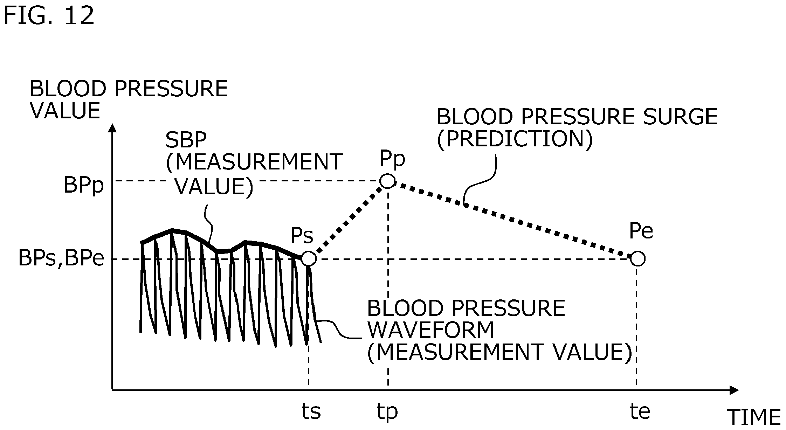

Next, the indicator extraction unit 50 predicts "what shape a surge in blood pressure will be if a surge occurs at the current point in time", based on the values of the SBP, the AI, and the BRS of the user at the current point in time (step 3903). Specifically, as shown in FIG. 12, the indicator extraction unit 50 performs processing to estimate the peak point Pp = (tp,BPp) and the end point Pe = (te,BPe) of a blood pressure surge, where a point Ps = (ts,BPs) is the SBP at the current point in time, given as the initial value. In the present example, the peak point Pp and the end point Pe are calculated according to the following equations.

Point in Time at Peak Point: tp = ts + k2

Blood Pressure are Peak Point: Pp = a1 × k2 + b

Point in Time at End Point: te = (BPp - BPs)/a2 + tp

Blood Pressure at End Point: BPe = BPs

Here, a rise rate a1 of the surge and a drop rate a2 of the surge are respectively calculated according to the following equations, using the AI and the BRS of the user at the current point in time.

Rise Rate of Surge: a1 = α × AI

Drop Rate of Surge: a2 = β × BRS

α and β are constants. A value b of blood pressure at the start point of the surge may be the blood pressure value BPs of the user at the current point in time, or the mean of values of blood pressure corresponding to the previous two or more heartbeats. k2 denotes the period of time from the start point to the peak of the surge, and is a constant. The values of α, β, and k2 may be obtained from an experiment performed on the subject or from case data, or set by the user based on the shapes of blood pressure surges that have occurred in the past.

Next, the indicator extraction unit 50 calculates the event occurrence risk based on the predicted shape of the blood pressure surge (step 3904). For example, the blood pressure value BPp at the peak point Pp of the blood pressure surge, the period of time te - ts from the start point Ps to the end point Pe of the blood pressure surge, the area of a triangle formed by the start point Ps, the peak point Pp, and the end point Pe, or a score obtained by combining them may be used as the event occurrence risk.

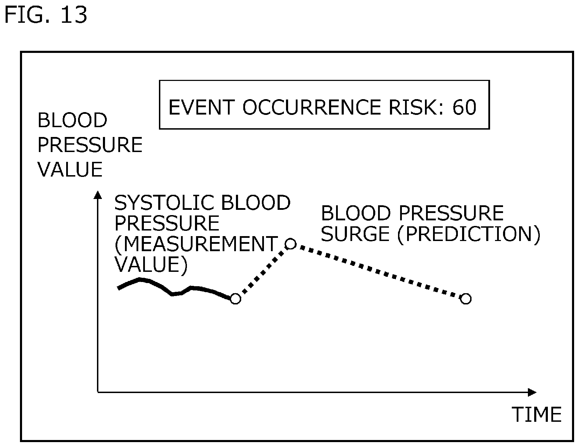

Next, the processing unit 51 displays the event occurrence risk calculated in step 3904, on a display device (step 3905). FIG. 13 shows an example of an information output screen. In the example shown in FIG. 13, the score of the event occurrence risk, the actual measurement value of the systolic blood pressure SBP, and the predicted blood pressure surge are displayed. Furthermore, if the event occurrence risk is greater than a threshold value (step 3906), the processing unit 51 informs the user of an increase in the event occurrence risk, by sounding an alarm and/or generating vibrations (step 3907). The processing performed in steps 3900 to 3907 is repeated for each heartbeat.

With the above-described configuration, it is possible to detect, in real time, an increase in the event occurrence risk. Also, when the risk has increased to a certain level, it is possible to allow the user to take countermeasures before an event occurs, by sounding an alarm and/or generating vibrations to inform the user. For example, if an increase in the risk is detected when the user is moving his/her body, the user may immediately assume a posture for rest, by sitting down, lying down, or the like. Also, if an increase in the risk caused by the occurrence of obstructive sleep apnea is detected, it is possible to prompt the user to wake up, by sounding an alarm and/or generating vibrations, or recommend the user to go to hospital.

The configurations according to the above-described embodiment and examples are no more than specific examples of configurations according to the present invention, and are not intended to limit the scope of the present invention. The present invention may employ various specific configurations without departing from the scope of the present invention defined by the claims.

INDEX TO THE REFERENCE NUMERALS

1 ... biological information analysis device, 2 ... measurement unit
10 ... biological information analysis system, 11 ... main body, 12 ... belt
20 ... blood pressure measurement unit, 21 ... body movement measurement unit, 22 ... environment measurement unit, 23 ... control unit, 24 ... input unit, 25 ... output unit, 26 ... communication unit, 27 ... storage unit
30 ... pressure sensor, 31 ... pressurizing mechanism, 300 ... pressure detection element 50 ... indicator extraction unit, 51 ... processing unit

**Claims**

1. A biological information analysis device (1) comprising:

   an indicator extraction unit (50) configured to extract, from data regarding blood pressure waveforms consecutively measured by a sensor (30) configured to be worn on a body part of a user and to be capable of noninvasively measuring a blood pressure waveform for each heartbeat, an indicator that indicates an event occurrence risk, which is the risk of an event occurring due to a change in blood pressure, based on a difference between an AI (Augmentation Index) of a measured blood pressure waveform and a reference AI and/or a difference between a BRS (Baroreflex sensitivity) of a measured blood pressure waveform and a reference BRS; and
   a processing unit (51) configured to perform processing that is based on the indicator, wherein
   the reference AI is a difference between a mean value of AI of occurred cases of the event and a standard deviation of AI of the occurred cases, and
   the reference BRS is a difference between a mean value of BRS of the occurred cases and a standard deviation of BRS of the occurred cases.

2. The biological information analysis device (1) according to claim 1,
   wherein the indicator extraction unit (50) is configured to predict a change in blood pressure based on characteristics of a blood pressure waveform of the user measured at the current point in time, assuming that a surge in blood pressure occurs at the current point in time, and calculate the indicator based on the result of prediction.

3. The biological information analysis device (1) according to claim 2,

wherein the indicator extraction unit (50) is configured to predict a change in blood pressure based on a SBP (Systolic Blood Pressure), an AI (Augmentation Index), and a BRS (Baroreflex sensitivity), which are characteristics of a blood pressure waveform, assuming that a surge in blood pressure occurs at the current point in time.

4. The biological information analysis device (1) according to any one of claims 1 to 3, wherein the processing unit (51) is configured to perform processing to provide information indicating that the event occurrence risk has increased, upon detecting an increase in the event occurrence risk based on the indicator.

5. A biological information analysis system (10) comprising:

a sensor (30) that is configured to be worn on a body part of a user and to be capable of non-invasively measuring a blood pressure waveform for each heartbeat; and
the biological information analysis device (1) according to any one of claims 1 to 4, the biological information analysis device (1) being configured to analyze biological information, using data regarding blood pressure waveforms consecutively measured by the sensor.

6. A program that causes a processor to function as the indicator extraction unit (50) and the processing unit (51) of the biological information analysis device (1) according to any one of claims 1 to 4.

7. A biological information analysis method comprising:

a step of extracting, by an indicator extraction unit (50), from data regarding blood pressure waveforms consecutively measured by a sensor (30) configured to be worn on a body part of a user and to be capable of non-invasively measuring a blood pressure waveform for each heartbeat, an indicator that indicates an event occurrence risk, which is the risk of an event occurring due to a change in blood pressure, based on a difference between an AI (Augmentation Index) of a measured blood pressure waveform and a reference AI and/or a difference between a BRS (Baroreflex sensitivity) of a measured blood pressure waveform and a reference BRS; and
a step of performing processing that is based on the indicator by means of a processing unit (51), wherein
the reference AI is a difference between a mean value of AI of occurred cases of the event and a standard deviation of AI of the occurred cases, and
the reference BRS is a difference between a mean value of BRS of the occurred cases and a standard deviation of BRS of the occurred cases.

**Patentansprüche**

1. Vorrichtung zur Analyse biologischer Informationen (1), umfassend:

eine Indikatorextrahiereinheit (50), die eingerichtet ist zum Extrahieren, aus Daten in Bezug auf Blutdruckwellenformen, fortlaufend gemessen mit einem Sensor (30), der so eingerichtet ist, dass er an einem Körperteil eines Benutzers getragen werden kann und dass er imstande ist, eine Blutdruckwellenform für jeden Herzschlag nichtinvasiv zu messen, eines Indikators, der ein Risiko des Eintretens eines Ereignisses anzeigt, welches das Risiko eines aufgrund einer Änderung des Blutdrucks eintretenden Ereignisses ist, basierend auf einer Differenz zwischen einem AI (Augmentationsindex) einer gemessenen Blutdruckwellenform und einem Referenz-AI und/oder einer Differenz zwischen einer BRS (Baroreflexsensitivität) einer gemessenen Blutdruckwellenform und einer Referenz-BRS; und
eine Verarbeitungseinheit (51), die eingerichtet ist zum Durchführen der Verarbeitung, die auf dem Indikator basiert, wobei
der Referenz-AI eine Differenz zwischen einem Mittelwert des AI aufgetretener Fälle des Ereignisses und einer Standardabweichung des AI der aufgetretenen Fälle ist und die Referenz-BRS eine Differenz zwischen einem Mittelwert der BRS der aufgetretenen Fälle und einer Standardabweichung der BRS der aufgetretenen Fälle ist.

2. Vorrichtung zur Analyse biologischer Informationen (1) nach Anspruch 1, wobei die Indikatorextrahiereinheit (50) eingerichtet ist zum Vorhersagen einer Veränderung des Blutdrucks, basierend auf Charakteristika einer zu dem aktuellen Zeitpunkt gemessenen Blutdruckwellenform des Benutzers, in der Annahme, dass zu dem aktuellen Zeitpunkt ein Anstieg des Blutdrucks erfolgt, und zum Berechnen des Indikators, basierend auf dem Ergebnis der Vorhersage.

3. Vorrichtung zur Analyse biologischer Informationen (1) nach Anspruch 2,
wobei die Indikatorextrahiereinheit (50) eingerichtet ist zum Vorhersagen einer Veränderung des Blutdrucks, basierend auf einem SBP (systolischen Blutdruck), einem AI (Augmentationsindex) und einer BRS (Baroreflexsensitivität), die Charakteristika einer Blutdruckwellenform sind, in der Annahme, dass zu dem aktuellen Zeitpunkt ein Anstieg des Blutdrucks erfolgt.

4. Vorrichtung zur Analyse biologischer Informationen (1) nach einem der Ansprüche 1 bis 3,
wobei die Verarbeitungseinheit (51) eingerichtet ist zum Durchführen der Verarbeitung, um Informationen, die anzeigen, dass sich das Risiko des Eintretens eines Ereignisses erhöht hat, nach dem Feststellen einer Erhöhung des Risikos des Eintretens eines Ereignisses, basierend auf dem Indikator, bereitzustellen.

5. System zur Analyse biologischer Informationen (10), umfassend:

einen Sensor (30), der so eingerichtet ist, dass er an einem Körperteil eines Benutzers getragen werden kann und dass er imstande ist, eine Blutdruckwellenform für jeden Herzschlag nichtinvasiv zu messen; und
die Vorrichtung zur Analyse biologischer Informationen (1) nach einem der Ansprüche 1 bis 4, wobei die Vorrichtung zur Analyse biologischer Informationen (1) eingerichtet ist zum Analysieren biologischer Informationen, unter Verwendung von Daten in Bezug auf fortlaufend mit dem Sensor gemessene Blutdruckwellenformen.

6. Programm, dass bewirkt, dass ein Prozessor als die Indikatorextrahiereinheit (50) und die Verarbeitungseinheit (51) der Vorrichtung zur Analyse biologischer Informationen (1) nach einem der Ansprüche 1 bis 4 dient.

7. Verfahren zur Analyse biologischer Informationen, umfassend:

einen Schritt des Extrahierens, mittels einer Indikatorextrahiereinheit (50), aus Daten in Bezug auf Blutdruckwellenformen, fortlaufend gemessen mit einem Sensor (30), der so eingerichtet ist, dass er an einem Körperteil eines Benutzers getragen werden kann und dass er imstande ist, eine Blutdruckwellenform für jeden Herzschlag nichtinvasiv zu messen, eines Indikators, der ein Risiko des Eintretens eines Ereignisses anzeigt, welches das Risiko eines aufgrund einer Änderung des Blutdrucks eintretenden Ereignisses ist, basierend auf einer Differenz zwischen einem AI (Augmentationsindex) einer gemessenen Blutdruckwellenform und einem Referenz-AI und/oder einer Differenz zwischen einer BRS (Baroreflexsensitivität) einer gemessenen Blutdruckwellenform und einer Referenz-BRS; und
einen Schritt des Durchführens der Verarbeitung, die auf dem Indikator basiert, mittels einer Verarbeitungseinheit (51), wobei
der Referenz-AI eine Differenz zwischen einem Mittelwert des AI aufgetretener Fälle des Ereignisses und einer Standardabweichung des AI der aufgetretenen Fälle ist und die Referenz-BRS eine Differenz zwischen einem Mittelwert der BRS der aufgetretenen Fälle und einer Standardabweichung der BRS der aufgetretenen Fälle ist.

## Revendications

1. Dispositif d'analyse d'informations biologiques (1) comprenant :

une unité d'extraction d'indicateur (50) configurée pour extraire, à partir des données concernant les formes d'onde de pression sanguine mesurées consécutivement par un capteur (30) configuré pour être porté sur une partie du corps d'un utilisateur et être capable de mesurer de manière non invasive une forme d'onde de pression sanguine pour chaque battement de cœur, un indicateur qui indique le risque d'occurrence d'un événement, qui est le risque d'un événement se produisant du fait d'un changement dans la pression sanguine, sur la base d'une différence entre un AI (indice d'augmentation) d'une forme d'onde de pression sanguine mesurée et un AI de référence et/ou une différence entre une BRS (sensibilité de baroréflexe) d'une forme d'onde de pression sanguine mesurée et une BRS de référence ; et
une unité de traitement (51) configurée pour effectuer un traitement qui est basé sur l'indicateur,
l'AI de référence étant une différence entre une valeur moyenne d'AI de cas s'étant produit de l'événement et un écart type d'AI des cas s'étant produit, et
la BRS de référence est une différence entre une valeur moyenne de BRS de cas s'étant produit et un écart type de BRS des cas s'étant produit.

2. Dispositif d'analyse d'informations biologiques (1) selon la revendication 1,

dans lequel l'unité d'extraction d'indicateur (50) est configurée pour prédire un changement de pression sanguine sur la base des caractéristiques d'une forme d'onde de pression sanguine de l'utilisateur mesurées au point dans le temps actuel, en supposant qu'un surgissement de pression sanguine se produit au point dans le temps actuel, et calculer l'indicateur sur la base du résultat de la prédiction.

3. Dispositif d'analyse d'informations biologiques (1) selon la revendication 2,
dans lequel l'unité d'extraction d'indicateur (50) est configurée pour prédire un changement de pression sanguine sur la base d'une SBP (pression du sang systolique), d'un AI (indice d'augmentation) et d'une BRS (sensibilité de baroréflexe), qui sont des caractéristiques d'une forme d'onde de pression sanguine, en supposant qu'un surgissement de pression sanguine se produit au point dans le temps actuel.

4. Dispositif d'analyse d'informations biologiques (1) selon l'une quelconque de revendications 1 à 3,
dans lequel l'unité de traitement (51) est configurée pour effectuer un traitement pour fournir des informations indiquant que le risque d'occurrence d'événement a augmenté, après la détection d'une augmentation du risque d'occurrence d'événement sur la base de l'indicateur.

5. Système d'analyse d'informations biologiques (10) comprenant :

un capteur (30) qui est configuré pour être porté sur une partie de corps d'un utilisateur et être capable de mesurer de manière non invasive une forme d'onde de pression sanguine pour chaque battement de cœur ; et le dispositif d'analyse d'informations biologiques (1) selon l'une quelconque des revendications 1 à 4, le dispositif d'analyse d'informations biologiques (1) étant configuré pour analyser des informations biologiques, en utilisant les données concernant les formes d'onde de pression sanguine mesurées consécutivement par le capteur.

6. Programme qui entraîne un processeur à fonctionner comme l'unité d'extraction d'indicateur (50) et l'unité de traitement (51) du dispositif d'analyse d'informations biologiques (1) selon l'une quelconque des revendications 1 à 4.

7. Procédé d'analyse d'informations biologiques comprenant :

une étape d'extraction, par une unité d'extraction d'indicateur (50), de données concernant les formes d'onde de pression sanguine mesurées consécutivement par un capteur (30) configuré pour être porté sur une partie de corps d'un utilisateur et être capable de mesurer de manière non invasive une forme d'onde de pression sanguine pour chaque battement de cœur, un indicateur qui indique le risque d'occurrence d'un événement, qui est le risque d'événement se produisant du fait d'un changement de pression sanguine, sur la base d'une différence entre un AI (indice d'augmentation) d'une forme d'onde de pression sanguine mesurée et un AI de référence et/ou une différence entre une BRS (sensibilité de baroréflexe) d'une forme d'onde de pression sanguine mesurée et une BRS de référence ; et
une étape de réalisation d'un traitement qui est basé sur l'indicateur au moyen d'une unité de traitement (51), l'AI de référence étant une différence entre une valeur moyenne d'AI de cas de l'événement s'étant produit et un écart type de l'AI des cas s'étant produit, et
la BRS de référence est une différence entre une valeur moyenne de BRS des cas s'étant produit et un écart type de BRS des cas s'étant produit.

FIG. 1

FIG. 2

EP 3 440 995 B1

FIG. 3

FIG. 4

FIG. 5

```
                  ┌──────────────┐                ┌──────────────┐
                  │   INDICATOR  │  INDICATOR     │  PROCESSING  │
MEASUREMENT  ───► │  EXTRACTION  │ ──────────►    │     UNIT     │ ───►
    DATA          │     UNIT     │                │              │
                  └──────────────┘                └──────────────┘
                        50                               51
```

FIG. 6

FIG. 7

```
                    ┌─────────────┐
                    │    START    │
                    └──────┬──────┘
                           │
                           ▼
        ┌─────────────────────────────────────┐
        │ ACQUIRE STATISTICS OF AI AND BRS     │  3600
        │ REGARDING OCCURRED CASES             │
        │ FROM CARDIOVASCULAR CASE DB          │
        └─────────────────┬───────────────────┘
                           │
                           ▼
        ┌─────────────────────────────────────┐
        │ ACQUIRE  BLOOD PRESSURE              │  3601
        │ WAVEFORM DATA                        │
        └─────────────────┬───────────────────┘
                           │
                           ▼
        ┌─────────────────────────────────────┐
        │ DETECT CHARACTERISTIC POINTS         │  3602
        │ OF BLOOD PRESSURE WAVEFORM           │
        └─────────────────┬───────────────────┘
                           │
                           ▼
        ┌─────────────────────────────────────┐
        │ CALCULATE AI AND BRS                 │  3603
        └─────────────────┬───────────────────┘
                           │
                           ▼
        ┌─────────────────────────────────────┐
        │ CALCULATE                            │  3604
        │ EVENT OCCURRENCE RISK                │
        └─────────────────┬───────────────────┘
                           │
                           ▼
        ┌─────────────────────────────────────┐
        │ DISPLAY EVENT OCCURRENCE RISK        │  3605
        └─────────────────┬───────────────────┘
                           │
                           ▼
                      3606
                    ◇─────────◇
                   ╱   RISK     ╲        NO
                  ◇ >THRESHOLD   ◇──────────┐
                   ╲   VALUE?   ╱           │
                    ◇─────────◇             │
                         │ YES              │
                         ▼                  │
        ┌─────────────────────────────┐     │
        │ NOTIFY USER OF              │ 3607 │
        │ INCREASE IN RISK           │      │
        └─────────────┬──────────────┘      │
                      │                      │
                      ▼                      │
                 ┌─────────┐                 │
                 │   END   │◄────────────────┘
                 └─────────┘
```

FIG. 8

DEGREE

AI RISK

MEAN
VALUE    USER'S AI

AI

REFERENCE AI
= MEAN VALUE − STANDARD DEVIATION

FIG. 9

EVENT OCCURRENCE RISK: 60

BLOOD
PRESSURE

TIME

FIG. 10

FIG. 11

```
                    ┌─────────────┐
                    │    START    │
                    └──────┬──────┘
                           │
          ┌────────────────▼────────────────┐
          │  ACQUIRE  BLOOD PRESSURE         │   3900
          │  WAVEFORM DATA                   │
          └────────────────┬────────────────┘
                           │
          ┌────────────────▼────────────────┐
          │  DETECT CHARACTERISTIC POINTS    │   3901
          │  OF BLOOD PRESSURE WAVEFORM      │
          └────────────────┬────────────────┘
                           │
          ┌────────────────▼────────────────┐
          │     CALCULATE AI AND BRS         │   3902
          └────────────────┬────────────────┘
                           │
          ┌────────────────▼────────────────┐
          │    PREDICT SHAPE OF SURGE        │   3903
          └────────────────┬────────────────┘
                           │
          ┌────────────────▼────────────────┐
          │        CALCULATE                 │   3904
          │  EVENT OCCURRENCE RISK           │
          └────────────────┬────────────────┘
                           │
          ┌────────────────▼────────────────┐
          │ DISPLAY EVENT OCCURRENCE RISK    │   3905
          └────────────────┬────────────────┘
```

RISK >THRESHOLD VALUE?   3906

NO

YES

NOTIFY USER OF INCREASE IN RISK   3907

END

FIG. 12

FIG. 13

EVENT OCCURRENCE RISK: 60

BLOOD PRESSURE VALUE

SYSTOLIC BLOOD PRESSURE (MEASUREMENT VALUE)

BLOOD PRESSURE SURGE (PREDICTION)

TIME

## REFERENCES CITED IN THE DESCRIPTION

**Patent documents cited in the description**

- JP 2008061824 A
- JP 2005532111 A
- US 2014276123 A1
- US 2015196209 A1
- US 20070021673 A1
- JP 2016082069 A
- JP 2016087003 A